# EUROPEAN PATENT SPECIFICATION

(11) **EP 4 541 325 B1**
(45) Date of publication and mention of the grant of the patent: **02.12.2026**
(21) Application number: 23204539.3
(22) Date of filing: 19.10.2023
(51) Int. Cl.: A61B 17/17, A61B 17/15, A61F 2/38, A61F 2/46, A61B 17/02

(54) **INSTRUMENT FOR KNEE ARTHROPLASTY PROCEDURE**
INSTRUMENT FÜR KNIEARTHROPLASTIE-EINGRIFF
INSTRUMENT POUR INTERVENTION D'ARTHROPLASTIE DU GENOU

(43) Date of publication of application: 23.04.2025
(73) Proprietor: Aesculap AG, 78532 Tuttlingen (DE)
(72) Inventor: Zouaghi, Housseyn, 52000 Chaumont (FR); Bork, Jana, 78315 Radolfzell (DE)
(74) Representative: DREISS Patentanwälte PartG mbB

(56) References cited:
- WO-A1-2012/004580
- CN-A- 107 233 148
- US-A1- 2017 340 458
- US-A1- 2019 380 721

## Description

The invention relates to a spacer instrument for use in a knee arthroplasty procedure. Such a spacer instrument allows evaluating an extension gap and/or flexion gap between a femur and a tibia or the respective implants.

US 2017/0340458 A1 and CN 107233148 A disclose an assembly of a plurality of tibial spacers which are rotatably attached to each other through a fastener, and which may be stacked onto each other, as needed, to achieve a desired height.

### Disclosure of the Invention

A first aspect of the description refers to a spacer instrument for use in a knee arthroplasty procedure, the spacer instrument comprising:
a spacer carrier device with a first spacer component comprising a first thickness and a second spacer component comprising a second thickness, the first thickness being different than the second thickness, each of the spacer components being configured to be temporarily positioned on top of a resected proximal portion of a tibia during knee arthroplasty,
and a rotative tibia component, wherein the rotative tibia component comprises a thickness corresponding to a thickness of trial tibia plateau and/or tibia implant, and wherein the rotative tibia component is rotatively attached to the spacer carrier device via a rotation mechanism, and the rotative tibia component is movable in a first position and a second position, wherein in the first position the first spacer component is aligned above the rotative tibia component and wherein in the second position the second spacer component is aligned above the rotative tibia component, such that the first spacer component or the second spacer component can be temporarily positioned on top of the resected proximal portion of a tibia with or without the rotative tibia component.

Positioning on top of means not necessarily directly positioning on the resected proximal portion. Each spacer component can be positioned for example on a trial tibia plateau attached to the resected tibia, or a tibia implant attached to the resected tibia or together with the rotative tibia component directly on the resected proximal portion.

The rotative tibia component comprises a thickness corresponding to a thickness of trial tibia plateau and/or tibia implant. Therefore, the rotative tibia component replaces the trial tibia plateau or the tibia implant at an early stage of the knee arthroplasty operation, before the trial tibia plateau or the tibia implant is attached to the resected tibia. Once the trial tibia plateau or the tibia implant is attached to the resected tibia, each spacer component can be positioned directly thereon without the rotative tibia component.

The advantage of the inventive spacer instrument is that the instrument can be used to evaluate the extension and/or flexion gap at any stage of the knee arthroplasty procedure, for example at an early, before the trial tibia plateau or the tibia implant is attached to the resected tibia, or at a later stage, once the trial tibia plateau or the tibia implant is attached to the resected tibia.

For example, each spacer component is a plate-type component having a substantially planar distal surface and a substantially planar proximal surface. The two spacer components are for example connected via a connecting part of the carrier device. The connecting part is for example a bar-type part. The rotative tibia component is for example attached to the connecting part via the rotation mechanism.

Advantageously, the rotation mechanism comprises a manually operable operating element, which allows unlocking of the rotative tibia component. It is required to manually actuate the rotation mechanism to unlock the rotative tibia component. Once the rotative tibia component is unlocked, the rotative tibia component is rotatable.

Advantageously, the first position and the second position is a locked position, and the rotative tibia component is rotatable between the first position and the second position upon unlocking of the rotative tibia component via the operating element. Upon unlocking of the rotative tibia component, the rotative tibia component can be rotated manually around a rotational axis.

In some exemplary embodiments, the rotation mechanism comprises a self-lock mechanism for automatically locking the rotative tibia component in the first position and in the second position. The self-lock mechanism is for example a snap-in mechanism, which locks automatically in the first and the second position. Once the rotative tibia component reaches the first or the second position, no manually actuation is required to lock the rotative tibia component in said position.

The rotative tibia component might be plate-type component having a substantially planar distal surface and a substantially planar proximal surface. The rotative tibia component might be a hollow body at least enveloped by the distal surface, the proximal surface and a lateral surface. According to an example, at least one of said surfaces might comprise one or more openings, for example through holes. Such openings might be beneficial for hygienic preparation and/or for material saving and/or weight saving.

According to further preferred embodiments, the rotative tibia component comprises a plate-type base with a substantially planar distal surface and at least a lateral wall protruding from the plate-type base in the proximal direction. The height of the lateral wall defines the thickness of the rotative tibia component. The plate-type base and or the lateral wall might comprise one or more openings, for example through holes. Such openings might be beneficial for hygienic preparation and/or for material saving and/or weight saving.

Advantageously, the spacer instrument comprises connection means for releasably connecting a femur cut compensation component. A femur cut compensation component allows for using the spacer instrument before or after distal resection of femur by adding the femur cut compensation component. According to an example, the connection means comprise a least one T-shaped groove, wherein the femur cut compensation component comprises a corresponding T-shaped member for engaging with the T-shaped groove. According to an example, each of the spacer components of the spacer instrument comprises a T-shaped groove.

Advantageously, the spacer instrument and/or the femur cut compensation component comprise mounting means for releasably mounting a femur cutting block including corresponding mounting means. According to an example, the femur cutting block provides a cutting guide for femur resection.

Advantageously, the spacer instrument comprises receiving means, in particular through holes, for releasably receiving an alignment rod. According to an example, the spacer instrument comprises two, in particular elongated holes. According to an example, the two long holes are placed on the connecting part of the carrier device each on one side of the rotation mechanism. The rotative tibia component might comprise corresponding receiving means, in particular a through hole. The alignment rod allows checking of the tibia mechanical axis alignment.

A second aspect of the description refers to a set of spacer instruments comprising at least two spacer instruments, each comprising a first spacer component comprising a first thickness and a second spacer component comprising a second thickness, the first thickness and the second thickness defining a pair of thicknesses for each spacer instrument, wherein the at least two spacer instruments of the set are differing by different pairs of thicknesses.

A third aspect of the description refers to a kit of parts comprising at least one spacer instrument according to the embodiments, and at least one femur cut compensation plate and/or a cutting block and/or an alignment rod and/or at least one further instrument. A further instrument might be for example a measurement block or sizing instrument.

Further advantageous embodiments are derivable from the following description of exemplary embodiments with reference to the drawings. In the drawings:
- Fig. 1: schematically depicts a spacer instrument positioned on top of a resected proximal portion of a tibia according to different stages of a knee arthroplasty procedure (a) and b));
- Fig. 2: schematically depicts a spacer instrument in different operating situations (a), b) and c));
- Fig. 3: schematically depicts a spacer instrument in an exploded view (a), a component of the spacer instrument (b) and detailed views of the spacer instrument (c), d) and e));
- Fig. 4: schematically depicts a component of the spacer instrument in different views (a) and b));
- Fig. 5: schematically depicts the component according to Fig. 4 attached to the spacer instrument in different views (a), b) and c));
- Fig. 6: schematically depicts a spacer instrument according to different stages of a knee arthroplasty procedure (a) and b)).

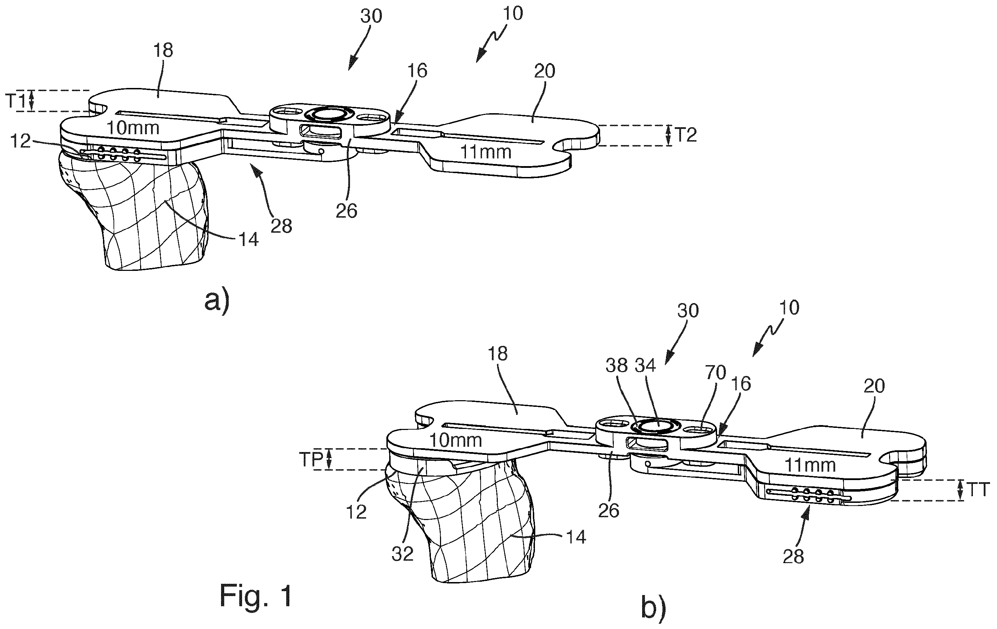

Fig. 1 schematically depicts a spacer instrument 10 for use in a knee arthroplasty procedure. According to the example, the spacer instrument positioned on top of a resected proximal portion 12 of a tibia 14.

The spacer instrument 10 comprises a carrier device 16 with a first spacer component 18 and a second spacer component 20. The first spacer component 18 comprises a first thickness T1 and the second spacer component 20 comprises a second thickness T2. The first thickness T1 and the second thickness T2 are defining a pair of thickness, wherein the first thickness T1 is different from the second thickness T2. According to an example, a pair of thickness can be chosen from the following pairs of thicknesses may be provided: a first pair with T1=10 mm and T2=11mm, a further pair with T1=12 mm and T2=14mm, a further pair with T1=16 mm and T2=18mm. According to a further example, T1 or T2 can be 22mm.

According to the example, the each spacer component 18, 20 is a plate-type component having a substantially planar distal surface 22 and a substantially planar proximal surface 24. Each of the spacer components 18, 20 is configured to be temporarily positioned on top of the resected proximal portion 12 of the tibia 14 during knee arthroplasty with the distal surface 22 facing the resected proximal portion 12 of the tibia 14.

The two spacer components 18, 20 are connected via a connecting part 26 of the carrier device 16. The connecting part 26 is for example a bar-type part.

The spacer instrument 10 comprises a rotative tibia component 28. The rotative tibia component 28 is rotatively attached to the spacer carrier device 16 via a rotation mechanism 30.

The rotative tibia component 28 is movable in a first position and a second position. In the first position, the first spacer component 18 is aligned above the rotative tibia component 28, see for example fig. 1 a). In the second position, the second spacer 20 component is aligned above the rotative tibia component 28, see for example fig. 1 b). This allows that each of the spacer components 18, 20 can be temporarily positioned on top of the resected proximal portion 12 of the tibia 14 with or without the rotative tibia component 28.

According to fig. 1 a), the first spacer component 18 is aligned above the rotative tibia component 28 and the first spacer component 18 and the rotative tibia component 28 are positioned together on top of the resected proximal portion 12 of the tibia 14. The rotative tibia component 28 replaces a trial tibia plateau 32 or a tibia implant at an early stage of the operation procedure. A thickness TT of the rotative tibia component 28 corresponds to a thickness TP of the trial tibia plateau 32 or the tibia implant, see Fig. 1 b).

According to fig. 1 b), the rotative tibia component 28 is moved into the second position and the second spacer component 20 is aligned above the rotative tibia component 28. The first spacer component 18 is positioned on top of the resected proximal portion 12 of the tibia 14 on a trial tibia plateau 32 or a tibia implant, which is attached to the resected tibia at a later stage of the operation procedure.

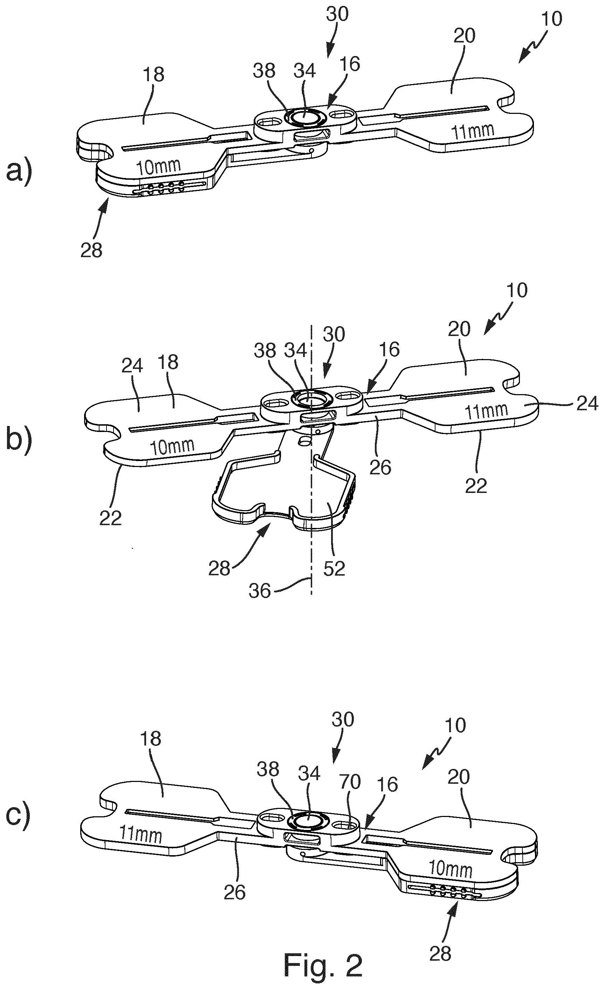

The rotation mechanism 30 comprises a manually operable operating element 34, which allows unlocking of the rotative tibia component. The operating element 34 is for example a button. It is required to manually actuate the rotation mechanism 30 via the operating element 34 to unlock the rotative tibia component 28. According to the example, manually actuation of the rotation mechanism 30 is done by manually pressing the operating element 34 in distal direction along the rotational axis 36. Once the rotative tibia component 28 is unlocked, the rotative tibia component is rotatable 28 around a rotation axis 36. The first position, see fig. 2 a), and the second position, see fig. 2 c) is a locked position. Upon unlocking of the rotative tibia component 28, the rotative tibia component 28 can be rotated manually around the rotational axis 36, see fig. 2 b).

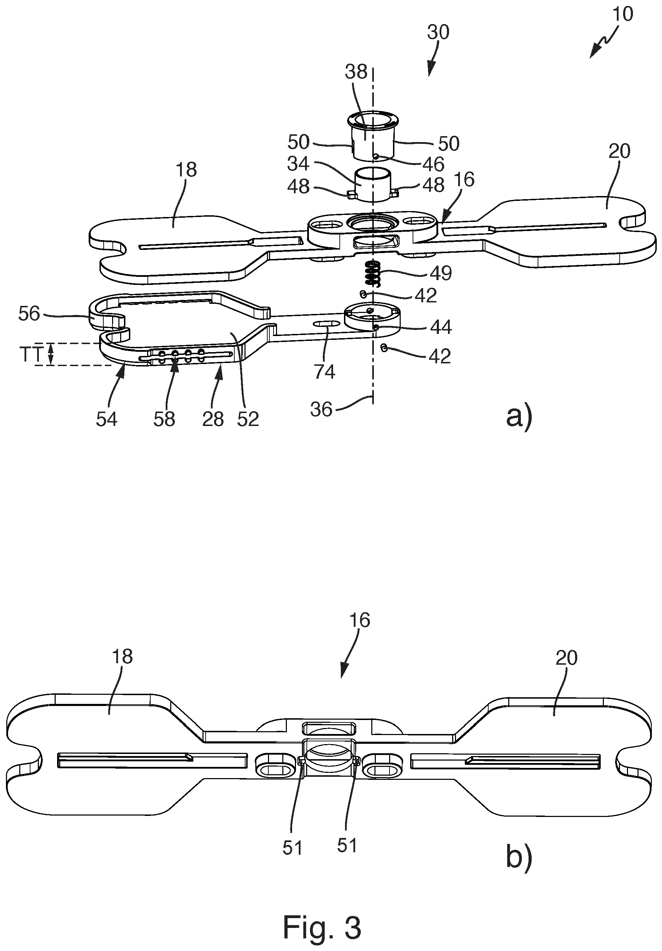

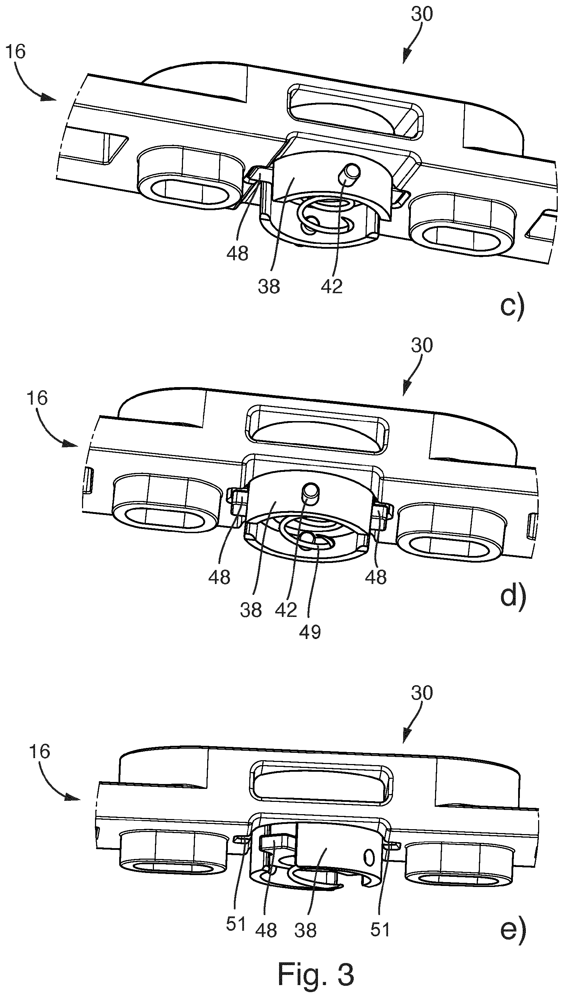

The rotation mechanism 30 is now explained with regard to fig. 3. In the assembled state, the operating element 34 is guided by a guiding part 38, which receives the operating element 34 and allows for moving the operating element 34 along the rotational axis 36 and for rotating the operating element 34 around the rotational axis 36. In the assembled state, the rotative tibia 28 plate is fixed to the guiding part 38. For example, the rotative tibia component 28 is fixed to the guiding part 38 via two pins 42 welded or glued into corresponding recesses 44, 46 of the rotative tibia component 28 and the guiding part 38.

The rotation mechanism 30 comprises a self-lock mechanism for automatically locking the rotative tibia component in the first position and in the second position. According to the example, the self-lock mechanism is realized via two protrusions 48 of the operating element 34 and a spring element 49. The two protrusions 48 are arranged in corresponding notches 50 of the guiding part 38 and the two protrusions extend through the notches 50 of the guiding part 38.

The spring element 49 is arranged between the rotative tibia component 28 and the operating element 34. The spring force acts on the rotative tibia component 28 and the operating element 34 and results in pressing the operating element 34 in the proximal direction. Once the rotative tibia component 28 reaches the first or the second position and the operating element 34 is released, the two protrusions 48 of the operating element 34 snap into corresponding pockets 51 of the carrier device 16, see fig. 3 b) and c). Thereby, the rotative tibia component 28 is locked via the rotation mechanism 30 together with the carrier device 16.

Therefore, the self-lock mechanism locks automatically in the first and the second position by releasing the operating element 34 and no manually actuation is required to lock the rotative tibia component in said position.

By pressing the operating element 34 in distal direction along the rotational axis 36, the protrusions 48 slide out of the pockets 51 of the carrier device 16, see fig. 3 d) and e), thereby allowing for rotating the rotative tibia component 28 manually around the rotational axis 36.

According the example, the rotative tibia component 28 comprises a plate-type base 52 with a substantially planar distal surface 54 and at least a lateral wall 56 protruding from the plate-type base 52 in the proximal direction. The height of the lateral wall 56 defines the thickness TT of the rotative tibia component 28. In the example, the plate-type base 52 and the lateral wall 56 comprise a number of openings 58, for example through holes, see fig. 3.

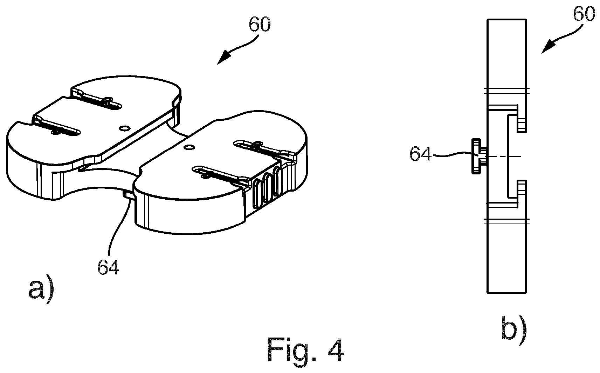

Fig. 4 depicts a femur cut compensation component 60. The femur cut compensation component 60 can be releasably connected to the spacer instrument 10, for example to the first and/or the second spacer component 18, 20. The femur cut compensation component 60 allows for using the spacer instrument before or after distal resection of femur by adding the femur cut compensation component.

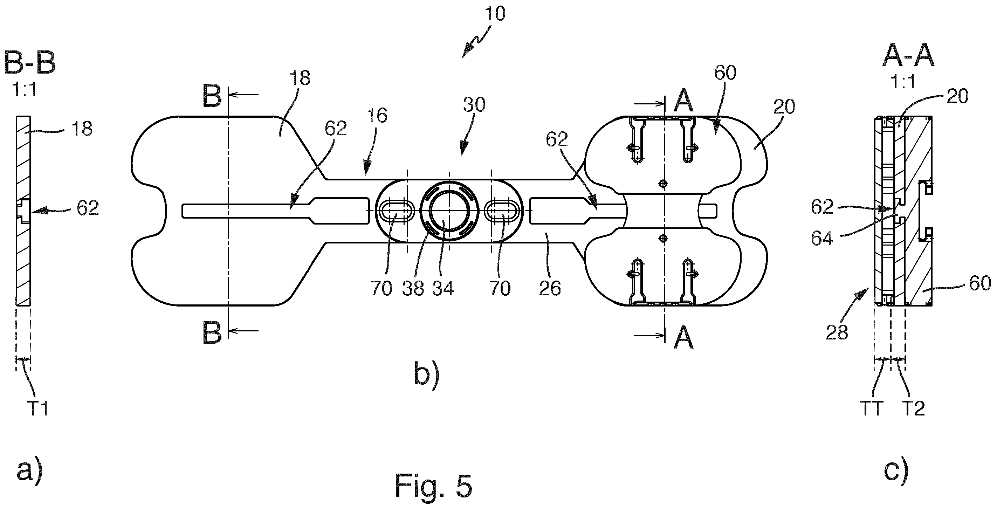

The spacer instrument 10 comprises connection means for releasably connecting the femur cut compensation component 60. According to the example, the connection means comprise a T-shaped groove 62 in each of the spacer components 18, 20, see fig. 5 a) to 5 c). The femur cut compensation component 60 comprises a corresponding T-shaped member 64 for engaging with one of the T-shaped groove 62, see fig. 4 a) and b).

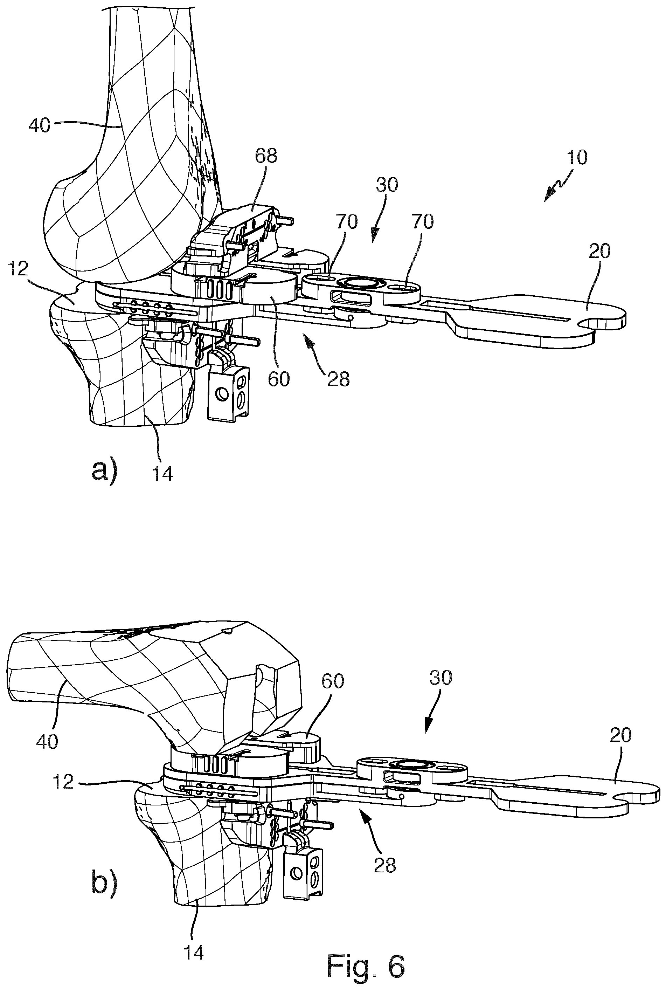

According to the example, the spacer instrument 10, in particular the femur cut compensation component 60, may comprise mounting means (not displayed) for releasably mounting further instruments, for example a femur cutting block 68 including corresponding mounting means. According to an example, the femur cutting block 68 provides a cutting guide for femur resection, see Fig. 6 a). Fig. 6 a) depicts a stage of the knee arthroplasty procedure before distal resection of femur 40. Fig. 6 b) depicts a stage of the knee arthroplasty procedure after distal resection of femur 40.

According to the example, the spacer instrument 10 comprises receiving means 70, in particular through holes, for releasably receiving an alignment rod (not displayed). According to the example, the spacer instrument 10 comprises two, in particular elongated, holes 70. According to an example, the two elongated holes 70 are placed on the connecting part 26 of the carrier device 16 each on one side of the rotation mechanism 30. The rotative tibia component 28 comprises corresponding receiving means 74, in particular a through hole. The alignment rod allows checking of the tibia 14 mechanical axis alignment. Further embodiments refer to a set of spacer instruments 10 comprising at least two spacer instruments 10 described with regard to figures 1 to 6. The at least two spacer instruments 10 each comprising a first spacer component 18 with a first thickness T1 and a second spacer component 20 with a second thickness T2, the first thickness T1 and the second thickness T2 defining a pair of thicknesses (T1, T2) for each spacer instrument 10, wherein the at least two spacer instruments 10 of the set are differing by different pairs of thicknesses (T1, T2).

Further embodiments refer to a kit of parts comprising at least one spacer instrument 10 described with regard to figures 1 to 6, and at least one femur cut compensation plate 60 and/or a cutting block 68 and/or an alignment rod.

The following method does not form part of the present invention and is described for illustrative purposes only.

A method for carrying out a knee arthroplasty procedure comprises the following steps:
In a step of the procedure, the proximal portion 12 of a tibia 14 is prepared by resecting the proximal portion.

In a step of the procedure, an extension gap and/or flexion gap between the femur and the tibia is evaluated.

The step of evaluating the extension gap and/or flexion gap might comprise positioning a spacer instrument 10, in particular a first spacer component 18 or a second spacer component 20 on the resected proximal portion 12. The first spacer component 18 or the second spacer component might be aligned above the rotative tibia component 28 such that the first spacer component 18 and the rotative tibia component 28 or the second spacer component 20 and the rotative tibia component 28 are positioned together on top of the resected proximal portion 12 of the tibia 14.

The step of evaluating the extension gap and/or flexion gap might comprise rotating the rotative tibia component 28 from the first position to the second position and/or from a second position to the first position via the rotation mechanism 30. Rotating the rotative tibia component 28 might comprise manually operating the operating element 34, which allows unlocking of the rotative tibia component 28 and manually rotating the rotative tibia component 28.

After rotating the rotative tibia component 28 from the first position to the second position and/or vice versa, the first spacer component 18 or the second spacer component 20 might be positioned on the resected proximal portion 12 together with the rotative tibia component 28.

In a step of the procedure, a trial tibia plateau 32 or a tibia implant might be attached to the proximal portion 12 of the tibia 14.

In a further step of the procedure, an extension gap and/or flexion gap between the femur and the tibia with the trial tibia plateau 32 or a tibia implant is evaluated using said spacer instrument 10. The first spacer component 18 or the second spacer component 20 might be positioned directly on the trial tibia plateau or a tibia implant without the rotative tibia component 28. Rotating of the rotative tibia component 28 might be carried out as described above.

## Claims

1. A spacer instrument (10) for use in a knee arthroplasty procedure, comprising:
a spacer carrier device (16) with a first spacer component (18) comprising a first thickness (T1) and a second spacer component (20) comprising a second thickness (T2), the first thickness (T1) being different than the second thickness (T2), each of the spacer components (18, 20) being configured to be temporarily positioned on top of a resected proximal portion (12) of a tibia (14) during knee arthroplasty, and a rotative tibia component (28), wherein the rotative tibia component (28) comprises a thickness corresponding to a thickness of trial tibia plateau and/or tibia implant, and wherein the rotative tibia component (28) is rotatively attached to the spacer carrier device (16) via a rotation mechanism (30), and the rotative tibia component (28) is movable in a first position and a second position, wherein in the first position the first spacer component (18) is aligned above the rotative tibia component (28) and wherein in the second position the second spacer component (20) is aligned above the rotative tibia component (28), such that the first spacer component (18) or the second spacer component (20) can be temporarily positioned on top of the resected proximal portion (12) of a tibia (14) with or without the rotative tibia component (28).

2. The spacer instrument (10) according to claim 1,
wherein each spacer component (18, 20) is a plate-type component having a substantially planar distal surface (22) and a substantially planar proximal surface (24) and/or the two spacer components are connected via a connecting part (26).

3. The spacer instrument (10) according to any of the preceding claims, wherein the rotation mechanism (30) comprises a manual operable operating element (34), which allows unlocking of the rotative tibia component (28).

4. The spacer instrument (10) according to claim 3,
wherein the first position and the second position is a locked position, and the rotative tibia component (28) is rotatable between the first position and the second position upon unlocking of the rotative tibia component (28) via the operating element (34).

5. The spacer instrument (10) according to any of the preceding claims, wherein the rotation mechanism (30) comprises a self-lock mechanism for automatically locking the rotative tibia component (28) in the first position and in the second position.

6. The spacer instrument (10) according to any of the preceding claims, wherein the rotative tibia component (28) comprises a plate-type base (52) with a substantially planar distal surface (54) and at least a lateral wall (56) protruding from the plate-type base (52) in the proximal direction.

7. The spacer instrument (10) according to any of the preceding claims, wherein the spacer instrument (10) comprises connection means (62) for releasably connecting a femur cut compensation component (60).

8. The spacer instrument (10) according to any of the preceding claims, wherein the spacer instrument (10) comprises receiving means (70), in particular through holes, for releasably receiving an alignment rod.

9. A set of spacer instruments (10) comprising at least two spacer instruments (10) according to any of the preceding claims, each comprising a first spacer component (18) comprising a first thickness (T1) and a second spacer component (20) comprising a second thickness (T2), the first thickness (T1) and the second thickness (T2) defining a pair of thicknesses (T1, T2) for each spacer instrument (10), wherein the at least two spacer instruments (10) of the set are differing by different pairs of thicknesses (T1, T2).

10. A kit of parts comprising at least one spacer instrument (10) according to any of the claims 1 to 8, and at least one femur cut compensation plate (60) and/or a cutting block (68) and/or an alignment rod and/or at least one further instrument.

## Patentansprüche

1. Abstandshalterinstrument (10) zur Verwendung in einem Kniearthroplastieverfahren, umfassend:
eine Abstandshalterträgervorrichtung (16) mit einer ersten Abstandshalterkomponente (18), umfassend eine erste Dicke (T1), und einer zweiten Abstandshalterkomponente (20), umfassend eine zweite Dicke (T2), wobei die erste Dicke (T1) sich von der zweiten Dicke (T2) unterscheidet, jede der Abstandshalterkomponenten (18, 20) konfiguriert ist, um während einer Kniearthroplastie vorübergehend oben auf einem resezierten proximalen Abschnitt (12) einer Tibia (14) positioniert zu werden, und einer drehbaren Tibiakomponente (28), wobei die drehbare Tibiakomponente (28) eine Dicke umfasst, die einer Dicke eines Probe-Tibiaplateaus und/oder Tibiaimplantats entspricht, und wobei die drehbare Tibiakomponente (28) an der Abstandshalterträgervorrichtung (16) über einen Drehmechanismus (30) drehbar angebracht ist und die drehbare Tibiakomponente (28) in eine erste Position und eine zweite Position bewegbar ist, wobei in der ersten Position die erste Abstandshalterkomponente (18) über der drehenden Tibiakomponente (28) ausgerichtet ist und wobei in der zweiten Position die zweite Abstandshalterkomponente (20) über der drehenden Tibiakomponente (28) ausgerichtet ist, derart, dass die erste Abstandshalterkomponente (18) oder die zweite Abstandshalterkomponente (20) temporär oben auf dem resezierten proximalen Abschnitt (12) einer Tibia (14) mit oder ohne die drehende Tibiakomponente (28) positioniert werden kann.

2. Abstandshalterinstrument (10) nach Anspruch 1, wobei jede Abstandshalterkomponente (18, 20) eine plattenartige Komponente ist, die eine im Wesentlichen planare distale Oberfläche (22) und eine im Wesentlichen planare proximale Oberfläche (24) aufweist, und/oder die zwei Abstandshalterkomponenten über ein Verbindungsteil (26) verbunden sind.

3. Abstandshalterinstrument (10) nach einem der vorstehenden Ansprüche, wobei der Drehmechanismus (30) ein manuell bedienbares Bedienelement (34) umfasst, das ein Entriegeln der drehenden Tibiakomponente (28) ermöglicht.

4. Abstandshalterinstrument (10) nach Anspruch 3, wobei die erste Position und die zweite Position eine verriegelte Position ist und die drehende Tibiakomponente (28) zwischen der ersten Position und der zweiten Position bei Entriegelung der drehenden Tibiakomponente (28) über das Bedienelement (34) drehbar ist.

5. Abstandshalterinstrument (10) nach einem der vorstehenden Ansprüche, wobei der Drehmechanismus (30) einen Selbstverriegelungsmechanismus zum automatischen Verriegeln der drehenden Tibiakomponente (28) in der ersten Position und in der zweiten Position umfasst.

6. Abstandshalterinstrument (10) nach einem der vorstehenden Ansprüche, wobei die drehbare Tibiakomponente (28) eine plattenartige Basis (52) mit einer im Wesentlichen planaren distalen Oberfläche (54) und mindestens eine Seitenwand (56) umfasst, die von der plattenartigen Basis (52) in der proximalen Richtung vorsteht.

7. Abstandshalterinstrument (10) nach einem der vorstehenden Ansprüche, wobei das Abstandshalterinstrument (10) Verbindungsmittel (62) zum lösbaren Verbinden einer Femurschnitt-Kompensationskomponente (60) umfasst.

8. Abstandshalterinstrument (10) nach einem der vorstehenden Ansprüche, wobei das Abstandshalterinstrument (10) Aufnahmemittel (70), insbesondere Durchgangslöcher, zum lösbaren Aufnehmen eines Ausrichtungsstabs umfasst.

9. Satz von Abstandshalterinstrumenten (10), umfassend mindestens zwei Abstandshalterinstrumente (10) nach einem der vorstehenden Ansprüche, jeweils umfassend eine erste Abstandshalterkomponente (18), umfassend eine erste Dicke (T1), und eine zweite Abstandshalterkomponente (20), umfassend eine zweite Dicke (T2), wobei die erste Dicke (T1) und die zweite Dicke (T2) ein Paar von Dicken (T1, T2) für jedes Abstandshalterinstrument (10) definieren, wobei sich die mindestens zwei Abstandshalterinstrumente (10) des Satzes durch unterschiedliche Paare von Dicken (T1, T2) unterscheiden.

10. Teilekit, umfassend mindestens ein Abstandshalterinstrument (10) nach einem der Ansprüche 1 bis 8 und mindestens eine Femurschnitt-Kompensationsplatte (60) und/oder einen Schneidblock (68) und/oder einen Ausrichtungsstab und/oder mindestens ein weiteres Instrument.

## Revendications

1. Instrument d'espacement (10) destiné à être utilisé dans une intervention d'arthroplastie du genou, comprenant :
un dispositif de support d'espacement (16) avec un premier composant d'espacement (18) comprenant une première épaisseur (T1) et un second composant d'espacement (20) comprenant une seconde épaisseur (T2), la première épaisseur (T1) étant différente de la seconde épaisseur (T2), chacun des composants d'espacement (18, 20) étant conçu pour être positionné temporairement sur une partie proximale réséquée (12) d'un tibia (14) pendant une arthroplastie du genou, et un composant tibial rotatif (28), dans lequel le composant tibial rotatif (28) comprend une épaisseur correspondant à une épaisseur de plateau tibial d'essai et/ou d'implant tibial, et dans lequel le composant tibial rotatif (28) est fixé de manière rotative au dispositif de support d'espacement (16) par l'intermédiaire d'un mécanisme de rotation (30), et le composant tibial rotatif (28) est mobile dans une première position et une seconde position, dans lequel, dans la première position, le premier composant d'espacement (18) est aligné au-dessus du composant tibial rotatif (28) et dans lequel, dans la seconde position, le second composant d'espacement (20) est aligné au-dessus du composant tibial rotatif (28), de sorte que le premier composant d'espacement (18) ou le second composant d'espacement (20) peut être positionné temporairement sur la partie proximale réséquée (12) d'un tibia (14) avec ou sans le composant tibial rotatif (28).

2. Instrument d'espacement (10) selon la revendication 1, dans lequel chaque composant d'espacement (18, 20) est un composant de type plaque ayant une surface distale sensiblement plane (22) et une surface proximale sensiblement plane (24) et/ou les deux composants d'espacement sont reliés par l'intermédiaire d'une partie de liaison (26).

3. Instrument d'espacement (10) selon l'une quelconque des revendications précédentes, dans lequel le mécanisme de rotation (30) comprend un élément d'actionnement actionnable manuellement (34) qui permet le déverrouillage du composant tibial rotatif (28).

4. Instrument d'espacement (10) selon la revendication 3, dans lequel la première position et la seconde position sont une position verrouillée, et le composant tibial rotatif (28) peut tourner entre la première position et la seconde position lors du déverrouillage du composant tibial rotatif (28) par l'intermédiaire de l'élément d'actionnement (34).

5. Instrument d'espacement (10) selon l'une quelconque des revendications précédentes, dans lequel le mécanisme de rotation (30) comprend un mécanisme d'autoverrouillage pour verrouiller automatiquement le composant tibial rotatif (28) dans la première position et dans la seconde position.

6. Instrument d'espacement (10) selon l'une quelconque des revendications précédentes, dans lequel le composant tibial rotatif (28) comprend une base de type plaque (52) avec une surface distale sensiblement plane (54) et au moins une paroi latérale (56) faisant saillie de la base de type plaque (52) dans la direction proximale.

7. Instrument d'espacement (10) selon l'une quelconque des revendications précédentes, dans lequel l'instrument d'espacement (10) comprend un moyen de liaison (62) pour relier de manière amovible un composant de compensation de coupe fémorale (60).

8. Instrument d'espacement (10) selon l'une quelconque des revendications précédentes, dans lequel l'instrument d'espacement (10) comprend un moyen de réception (70), en particulier des trous traversants, pour recevoir de manière amovible une tige d'alignement.

9. Ensemble d'instruments d'espacement (10) comprenant au moins deux instruments d'espacement (10) selon l'une quelconque des revendications précédentes, chacun comprenant un premier composant d'espacement (18) comprenant une première épaisseur (T1) et un second composant d'espacement (20) comprenant une seconde épaisseur (T2), la première épaisseur (T1) et la seconde épaisseur (T2) définissant une paire d'épaisseurs (T1, T2) pour chaque instrument d'espacement (10), dans lequel les au moins deux instruments d'espacement (10) de l'ensemble diffèrent par des paires d'épaisseurs (T1, T2) différentes.

10. Kit de pièces comprenant au moins un instrument d'espacement (10) selon l'une quelconque des revendications 1 à 8, et au moins une plaque de compensation de coupe fémorale (60) et/ou un bloc de coupe (68) et/ou une tige d'alignement et/ou au moins un autre instrument.
